# EUROPEAN PATENT APPLICATION

(11) **EP 0 763 521 A2**
(43) Date of publication of application: **19.03.1997**
(21) Application number: 96305993.6
(22) Date of filing: 16.08.1996
(51) Int. Cl.: C07C 211/62, C07C 209/86, C07D 209/48, C07B 41/04, C07B 63/00

(54) **Method of recycling phase transfer catalyst for displacement reactions**

(30) Priority: 21.08.1995 US 517311
(71) Applicant: GENERAL ELECTRIC COMPANY, Schenectady, NY 12345 (US)
(72) Inventor: Haitko, Deborah Ann, Schenectady, New York 12309 (US); Dellacoletta, Brent Allen, Dunbar, West Virginia 25064 (US)
(74) Representative: Szary, Anne Catherine, Dr.

(57) **Abstract**

Quatemary salts, employed as phase transfer catalysts in reactions of salts of dihydroxyaromatic compounds with substituted aromatic compounds such as halo- or nitro-phthalimides in non-polar solvents such as toluene, are recovered by washing the solution with water or aqueous base and extracting the wash liquids with a chlorinated alkane such as methylene chloride. It is often preferred to contact the extracts with an aqueous alkali metal bromide solution prior to recovery of the quatemary salt, thus converting all anion in said salt to bromide.

## Description

### Background of the Invention

This invention relates to the reaction of dihydroxyaromatic compound salts with substituted aromatic compounds, and more particularly to the recovery of phase transfer catalysts used in such a reaction.

Various displacement reactions of salts of dihydroxyaromatic compounds with substituted aromatic compounds are known. Most often, the substituents on the substituted aromatic compounds are halide (especially chloride or bromide) or nitro radicals, and the dihydroxyaromatic compound salts are alkali metal and especially sodium salts. Reactions of this type include the formation of bisetherimides (intermediates in polyetherimide production) from substituted N-alkylphthalimides, the direct production of polyetherimides by the reaction of substituted bis(arylimido)alkanes with bisphenol salts, and the preparation of polyethersulfones and polyetherketones by the reaction of bis(haloaryl) ketones and sulfones with dihydroxyaromatic compound salts.

Early in the development of reactions of this type, they were generally conducted in dipolar aprotic solvents. The trouble and expense attending the use of such solvents inhibited the use of such reactions in commercial processes. Commercial use became much more practical, however, with the discovery that these reactions could be conducted in non-polar organic solvents with the use of phase transfer catalysts, typically quatemary salts as represented by tetraalkylammonium, tetraalkylphosphonium and hexaalkylguanidinium halides and their analogs. It was then possible to isolate the product of the displacement reaction by conventional methods such as anti-solvent precipitation, or to use the solution directly in further reactions.

A sizeable expense in conducting reactions in this way is the cost of the phase transfer catalyst. It is normally lost after one use. Recovery by concentration of the organic solution, followed by recycle, has been reported, but as a practical matter so little can be recovered in this way that the expense attributable to the lost material is still high.

One option for recycle of phase transfer catalyst, as described in U.S. Patent 4,520,204, is washing of the organic solution with water and/or dilute aqueous alkali, followed by extraction of the aqueous washings with o-dichlorobenzene in which the phase transfer catalyst is soluble. However, o-dichlorobenzene is a nonvolatile material and the phase transfer catalyst can thus be recycled only in solution therein; therefore, it is of little or no use unless the reaction solvent is o-dichlorobenzene.

A further conceptual obstacle to recycle of phase transfer catalyst is presented when a non-bromo-substituted aromatic compound (e.g., a chloro or nitro compound) is employed in the displacement reaction. The most effective and convenient phase transfer catalysts are quatemary bromides, and they are converted (at least in part) by displacement of other substituents to the corresponding chlorides or nitrites. The latter are not as effective as phase transfer catalysts, and are usually obtained as dark oils of poor or questionable purity.

It is of interest, therefore, to develop methods for recovery of quatemary salts which facilitate their recycle as phase transfer catalysts of high efficiency.

### Summary of the Invention

The present invention is based on the discovery of a class of solvents which is particularly useful for recovery of quaternary phase transfer catalysts. In addition, a procedure has been developed for conversion of impure quatemary chlorides and nitrites into relatively pure quatemary bromides which are highly effective as phase transfer catalysts upon recycle.

One aspect of the invention is a method for recovering quatemary salt from a mixture resulting from the reaction of at least one salt of a dihydroxyaromatic compound with at least one halo- or nitro-substituted aromatic compound in solution in a non-polar organic solvent and in the presence of said quatemary salt as a phase transfer catalyst, which comprises (A) washing said solution with a neutral or basic aqueous medium and (B) removing said quaternary salt from the wash liquid by extraction with at least one chlorinated alkane.

Another aspect is a method as previously described which further comprises (C) contacting the chlorinated alkane extract product of step B with an aqueous alkali metal bromide solution of at least about 2M concentration.

### Detailed Description; Preferred Embodiments

The salts of dihydroxy-substituted aromatic hydrocarbons which are employed in the present invention are typically alkali metal and preferably sodium or potassium salts. Sodium salts are especially preferred by reason of their availability and relatively low cost.

Suitable dihydroxy-substituted aromatic hydrocarbons include those having the formula

(I) HO-A¹-OH ,

wherein A¹ is a divalent aromatic hydrocarbon radical. Suitable A¹ radicals include m-phenylene, p-phenylene, 4,4'-biphenylene, 4,4'-bi(3,5-dimethyl)phenylene, 2,2-bis(4-phenylene)propane and similar radicals such as those which correspond to the dihydroxy-substituted aromatic hydrocarbons disclosed by name or formula (generic or specific) in U.S. Patent 4,217,438.

The A¹ radical preferably has the formula

(II) -A²-Y-A³- ,

wherein each of A² and A³ is a monocyclic divalent aromatic hydrocarbon radical and Y is a bridging hydrocarbon radical in which one or two atoms separate A² from A³. The free valence bonds in formula II are usually in the meta or para positions of A² and A³ in relation to Y. Compounds in which A¹ has formula II are bisphenols, and for the sake of brevity the term "bisphenol" is sometimes used herein to designate the dihydroxy-substituted aromatic hydrocarbons; it should be understood, however, that non-bisphenol compounds of this type may also be employed as appropriate.

In formula II, the A² and A³ values may be unsubstituted phenylene or hydrocarbon-substituted derivatives thereof, illustrative substituents (one or more) being alkyl and alkenyl. Unsubstituted phenylene radicals are preferred. Both A² and A³ are preferably p-phenylene, although both may be o- or m-phenylene or one o- or m-phenylene and the other p-phenylene.

The bridging radical, Y, is one in which one or two atoms, preferably one, separate A² from A³. Illustrative radicals of this type are methylene, cyclohexylmethylene, 2-[2.2.1]bicycloheptylmethylene, ethylene, isopropylidene, neopentylidene, cyclohexylidene, cyclopentadecylidene, cyclododecylidene and adamantylidene; gem-alkylene (alkylidene) radicals are preferred. Also included, however, are unsaturated radicals. For reasons of availability and particular suitability for the purposes of this invention, the preferred radical of formula II is the 2,2-bis(4-phenylene)propane radical, which is derived from bisphenol A and in which Y is isopropylidene and A² and A³ are each p-phenylene.

Spiro(bis)indane bisphenols may also be employed. They include 6,6'-dihydroxy-3,3,3',3'-tetramethylspiro(bis)indane and its substituted analogs.

The substituted aromatic compounds employed in the present invention may have one of the formulas

(III) Z(A⁴-X)₂

and 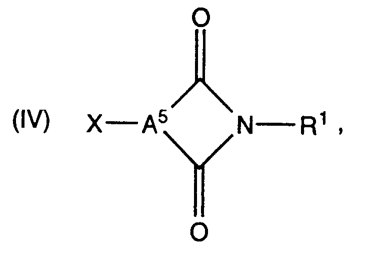 wherein Z is an activating radical, A⁴ is a divalent aromatic radical, A⁵ is a trivalent aromatic radical, X is fluoro, chloro, bromo or nitro and R¹ is a C₁₋₈ primary or secondary alkyl radical. The A⁴⁻⁵ radicals are normally C₆₋₁₀ radicals, preferably monocyclic and preferably free from electron-withdrawing substituents other than Z in the case of A⁴. Unsubstituted C₆ aromatic radicals are especially preferred.

The Z radical is usually a divalent electron-withdrawing group. Examples are carbonyl, carbonylbis(arylene), sulfone, bis(arylene) sulfone, benzo-1,2-diazine and azoxy. Thus, the moiety -A⁴-Z-A⁴- may be a bis(arylene) sulfone, bis(arylene) ketone, bis(arylene)benzo-1,2-diazine or bis(arylene)azoxy radical and especially one in which A⁴ is p-phenylene.

Also included are compounds in which -A⁴-Z-A⁴- is a bis(ether imide) radical, illustrated by those of the formula 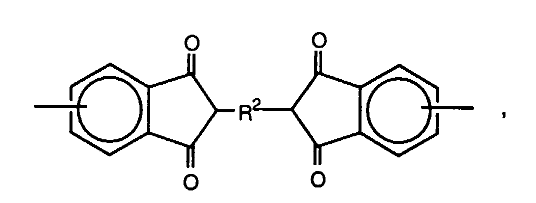 wherein R² is a C₆₋₂₀ divalent aromatic hydrocarbon or halogenated hydrocarbon radical, a C₂₋₂₀ alkylene or cycloalkylene radical, a C₂₋₈ bis(alkylene-terminated) polydiorganosiloxane radical or a divalent radical of the formula 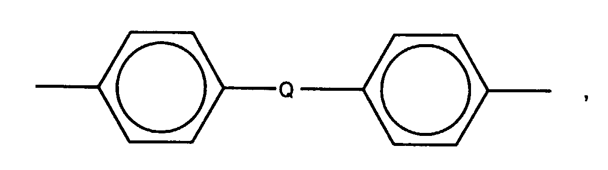 in which Q is -C₂H₄-, -O-, -CO-, -SO₂- or -C(CF₃)- or a covalent bond. Most often, R² is at least one of m-phenylene, p-phenylene, 4,4'-oxybis(phenylene) and

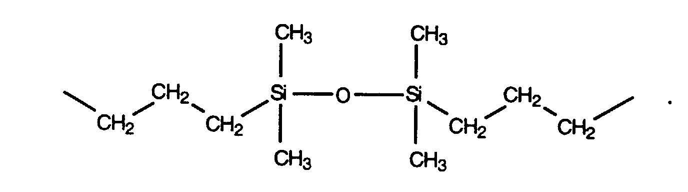

Also present in the substituted aromatic compound of formula III or IV are one or more displaceable X radicals which may be fluoro, chloro, bromo or nitro. In most instances, chloro, bromo or nitro atoms are preferred by reason of the relative availability and effectiveness of the compounds containing them.

Among the particularly preferred substituted aromatic compounds of formula III are bis(4-fluorophenyl) sulfone and the corresponding chloro compound, bis(4-fluorophenyl) ketone and the corresponding chloro compound, and 1,3- and 1,4-bis[N-(4-fluorophthalimido)]benzene and 4,4'-bis[N-(4-fluorophthalimido)]phenyl ether and the corresponding chloro, bromo and nitro compounds. Generally, however, compounds of formula IV, particularly the bromo-, nitro- and chloro-N-alkylphthalimides and especially the 4-substituted N-methylphthalimides, are preferred.

A third material employed according to the present invention is a non-polar solvent. Suitable solvents of this type include toluene, xylene, chlorobenzene, o-dichlorobenzene, dichlorotoluene and 1,2,4-trichlorobenzene. Toluene and xylene, and especially toluene, are preferred.

A quatemary salt is employed as a phase transfer catalyst. Suitable quaternary salts include tetraalkylammonium salts such as tetraethylammonium bromide, tetraethylammonium acetate and tetrabutylammonium bromide; bis(trialkyl)alkylenediammonium salts such as bis(tri-n-butyl)-1,6-hexylenediamonium dibromide; hexaalkylguanidinium salts or their heterocyclic analogs such as hexaethylguanidinium bromide and tris(piperidino)guanidinium bromide; and quatemary phosphonium salts such as tetraphenylphosphonium bromide. As a general rule, the quaternary ammonium and guanidinium salts having bromide or chloride as the anion are preferred by reason of their particular suitability.

The displacement reaction generally employs about 2 moles of substituted aromatic compound per mole of dihydroxyaromatic compound salt and is conducted at a temperature in the range of about 100-150°C. The quatemary salt is generally present in the amount of about 0.005-2.0 equivalents, preferably about 0.1-0.5 equivalent, per equivalent of hydroxyaromatic compound salt.

According to the present invention, the reaction mixture is washed, subsequent to the reaction between the hydroxyaromatic compound salt and the substituted aromatic compound, with a neutral or basic aqueous medium, typically water or dilute aqueous alkali up to about 0.5M and preferably up to about 0.25M. Suitable alkaline materials include sodium hydroxide, sodium carbonate, potassium hydroxide and potassium carbonate, with sodium hydroxide generally being preferred.

The washing temperature is ordinarily in the range of about 50-100°C. In most instances, more than one wash is preferred to maximize removal of phase transfer catalyst; two washes are usually sufficient.

Those skilled in the art will readily be able to determine whether washing with water or with dilute alkali is preferred in any particular instance. In general, it has been found that recovery of a hexaalkylguanidinium salt is facilitated by washing with aqueous alkali. On the other hand, recovery of a tetraalkylammonium salt may be facilitated by washing with water since some degree of decomposition of the tetraalkylammonium compound is frequently noted with alkaline washing.

A major proportion of the quatemary salt is removed from the organic layer in the wash step or steps. It may then be recovered from the aqueous medium by extraction with at least one chlorinated alkane.

Suitable chlorinated alkanes include methylene chloride, chloroform and 1,2-dichloroethane. The preferred chlorinated alkanes are those having high volatilities, as exemplified by boiling points up to about 75°C; methylene chloride is generally most preferred. It is frequently found that at least two such extractions are effective to recover a very high proportion of the quaternary salt. Separate extraction of each portion of wash liquid or combination of all portions followed by a single extraction or series of extractions may be employed.

When the substituted aromatic compound is a bromo compound, the quaternary salt recovered in the above-described procedure is the bromide, which is often suitable for direct recycle and use as a phase transfer catalyst in further iterations of the reaction. However, especially if the aromatic substituent is nitro or chloro the quaternary salt may be a mixture of bromide and nitrite or chloride, often with entrained impurities which can result in decreased catalytic activity.

Therefore, a highly preferred additional step when the aromatic substituent is other than bromo is to convert all anion in the extracted quatemary salt to bromide ion. This is conveniently achieved by contacting the extract with an aqueous alkali metal bromide solution. Said solution should generally have a concentration of at least about 2M. Saturated solutions are often preferred. The amount of bromide is not critical, provided that a stoichiometric excess with respect to quatemary salt is employed.

The conditions of the extraction and optional contact with alkali metal bromide solution are not critical. Said operations are typically conducted at a temperature in the range of about 20-40° and preferably about 20-30°C.

Following completion of the method of this invention, the product of the displacement reaction is present in the organic reaction solvent in a form which may be employed for further reactions, or from which product such as polymer may be recovered by conventional means. Recovered phase transfer catalyst is present in solution in the chlorinated alkane or, in the case of treatment with aqueous alkali metal bromide, in the resulting aqueous phase. If the latter, it may be extracted with a further portion of chlorinated alkane. Removal of the chlorinated alkane, typically by evaporation, yields phase transfer catalyst which may be recycled.

The method of this invention is illustrated by the following examples.

### Example 1

A mixture of 342 g (1.5 moles) of bisphenol A, 237 g of 50.62% aqueous sodium hydroxide solution and 600 ml of water was heated under reflux in a nitrogen atmosphere for 15 minutes. The solution of bisphenol A disodium salt was cooled to 85°C and 1500 ml of toluene was added. The mixture was heated under reflux for 9 hours, with removal of all water by azeotropic distillation. Further distillation was performed to remove 450 ml of toluene, after which 24.2 g (50 millimoles) of tetra-n-butylammonium bromide and 618.6 g (3 moles) of 4-nitro-N-methylphthalimide were added. The mixture was heated under reflux for 1 hour and diluted with 500 ml of toluene. Analysis showed formation of the desired 2,2-bis[4-(3,4-dicarboxyphenoxy)phenyl]propane bis-N-methylimide.

The toluene solution was then divided into two portions of equal volume. One portion was washed with two 600-ml portions of water at 75°C. The combined washings were cooled to room temperature, filtered and extracted with five 250-ml portions of methylene chloride. The methylene chloride extracts were filtered through anhydrous magnesium sulfate and vacuum stripped to afford 8.5 g of a dark yellow oil.

A 1-g sample of this oil was dissolved in about 20 ml of a 2.4M aqueous sodium bromide solution. The solution was filtered and the filtrate was further filtered through activated charcoal and extracted with three 20-ml portions of methylene chloride. Upon stripping of the methylene chloride, there was obtained 0.92 g of white, crystalline tetra-n-butylammonium bromide, for an overall recovery of 65%.

The recovered product was highly effective as a phase transfer catalyst in the reaction of bisphenol A disodium salt with 4-nitro-N-methylphthalimide. A control product obtained by a similar procedure but excluding the sodium bromide step was also effective but produced a lower yield under comparable conditions.

### Example 2

The second portion of the toluene solution from Example 1 was heated to about 75°C and washed with two 600-ml portions of 1% aqueous sodium hydroxide solution. Extraction with methylene chloride followed by treatment of a 1-g sample with aqueous sodium bromide solution, in accordance with Example 1, yielded 0.45 g of tetra-n-butylammonium bromide, for an overall recovery of 37%.

### Example 3

A mixture of 3.83 g (14 mmol) of the disodium salt of bisphenol A, 6.76 g (28 mmol) of 4-bromo-N-methylphthalimide, 400 mg of hexaethylguanidinium bromide, 574 mg of 1,3,5-triphenylbenzene as an intemal standard and 6 ml of toluene was heated in an oil bath at 150°C until the displacement reaction was complete as demonstrated by high pressure liquid chromatography. The temperature was then lowered to 80°C and the mixture was diluted with additional toluene to 22% solids.

The toluene solution was washed at 80°C with three 6-ml portions of a 0.8% aqueous sodium hydroxide solution; the mixture was vigorously stirred for 15 minutes during each washing, after which the aqueous layer was removed. Each sodium hydroxide washing was separately extracted with 7 ml of methylene chloride, after which the extracts were separately vacuum stripped. The first wash yielded 62%, the second wash 25% and the third wash 3% of the phase transfer catalyst present in the original reaction mixture.

### Example 4

A reaction was run by a procedure similar to that of Example 3, on a level of 11 mmol of bisphenol A salt and employing 4-nitro-N-methylphthalimide. Following completion of the reaction, the toluene reaction mixture was washed twice with 1% aqueous sodium hydroxide solution in an organic to aqueous ratio of 4:1. The two portions of washings were separately extracted with methylene chloride, and the two extracts were combined to produce a solution containing 90% of hexaethylguanidinium salt based on the amount originally employed. It was converted to hexaethylguanidinium bromide by dissolution in satuated aqueous sodium bromide solution, and the hexaethylguanidinium bromide was then recovered by extraction with methylene chloride followed by vacuum stripping.

## Claims

1. A method for recovering quaternary salt from a mixture resulting from the reaction of at least one salt of a dihydroxyaromatic compound with at least one halo- or nitro-substituted aromatic compound in solution in a non-polar organic solvent and in the presence of said quatemary salt as a phase transfer catalyst, which comprises (A) washing said solution with a neutral or basic aqueous medium and (B) removing said quatemary salt from the wash liquid by extraction with at least one chlorinated alkane.

2. A method according to claim 1 wherein the organic solvent is toluene or xylene.

3. A method according to claim 1 or claim 2 wherein the quaternary salt is a tetraalkylammonium, bis(trialkyl)alkylene diammonium or hexaalkylguanidinium salt.

4. A method according to claim 3 wherein the quatemary salt is a bromide or chloride.

5. A method according to any preceding claim wherein the substituted aromatic compound is chloro-, bromo- or nitro-substituted.

6. A method according to any preceding claim wherein the chlorinated alkane is methylene chloride.

7. A method according to any preceding claim which further comprises (C) contacting the chlorinated alkane extract product of step B with an aqueous alkali metal bromide solution of at least about 2M concentration.

8. A method according to claim 7 wherein contact with the alkali metal bromide solution is at a temperature in the range of about 50-100°C.

9. A method according to any preceding claim wherein step A is performed at least twice.

10. A method for recovering quatemary salt from a mixture resulting from the reaction of at least one salt of a dihydroxyaromatic compound with at least one bromo-, chloro- or nitro-substituted N-alkylphthalimide in toluene or xylene as solvent and in the presence of quatemary salt as a phase transfer catalyst, which comprises (A) washing said solution with water or aqueous sodium hydroxide and (B) removing said quaternary salt from the wash liquid by extraction with methylene chloride.

11. A method according to claim 10 wherein the phase transfer catalyst is a tetraalkylammonium bromide and the aqueous medium is water or wherein the quaternary salt is a hexaalkylguanidinium bromide and the aqueous medium is sodium hydroxide solution.

12. A method according to claim 10 or claim 11 which further comprises (C) contacting the methylene chloride extract product of step B with sodium bromide solution of at least about 2M concentration.
